# EUROPEAN PATENT APPLICATION

(11) **EP 4 083 205 A1**
(43) Date of publication of application: **02.11.2022**
(21) Application number: 20906126.6
(22) Date of filing: 24.12.2020
(51) Int. Cl.: C12N 15/11, C12Q 1/6869

(54) **DOUBLE-STRANDED NUCLEIC ACID MOLECULES AND METHOD FOR REMOVING GLASS ADAPTOR IN DNA LIBRARY BY MEANS OF SAME**

(30) Priority: 26.12.2019 KR 20190175696
(71) Applicant: Eone Diagnomics Genome Center Co., Ltd., Incheon 22014 (KR)
(72) Inventor: MIN, Na Young, Incheon 22014 (KR); BAE, Jin-Sik, Incheon 22014 (KR); LEE, Min Seob, Incheon 22014 (KR); SHIN, Shang Cheol, Incheon 22014 (KR)
(74) Representative: Bryn Aarflot AS
(86) International application number: PCT/KR2020/019070
(87) International publication number: WO 2021/133088

(57) **Abstract**

The present invention relates to a method for removing free adapters in a DNA library using a double-stranded nucleic acid molecule and a restriction enzyme, and more specifically, to a method for removing free adapters in a DNA library for next generation sequencing (NGS) using a double-stranded nucleic acid molecule including a type IIs restriction enzyme recognition site and a complementary sequence thereof, and a type IIs restriction enzyme.

## Description

### TECHNICAL FIELD

This application claims the priority of Korean Patent Application No. 10-2019-0175696, filed on December 26, 2019, the entirety of which is a reference of the present application.

The present invention relates to a method for removing free adapters generated in a process of constructing an NGS library using double-stranded nucleic acid molecules and a restriction enzyme, and more specifically, to a method for removing free adapters in a DNA library for next generation sequencing (NGS) using a double-stranded nucleic acid molecule, which comprises a type IIs restriction enzyme recognition site and a complementary sequence thereof, and a type IIs restriction enzyme.

### BACKGROUND ART

A genome refers to all genetic information of an organism. For sequencing of a genome of any one individual, various technologies such as a DNA chip, next generation sequencing (NGS), next next generation sequencing (NNGS), and the like have been developed. The NGS has been widely used for research and diagnosis. The NGS varies depending on a type of equipment, but may be largely divided into three steps: sample collection, library construction, and nucleic acid sequencing. After nucleic acid sequencing, various genetic information such as genetic mutation and the like may be confirmed based on produced sequencing data.

With improvement of next generation sequencing technology, sequencing rate and data output are greatly increased, so that a sample throughput of a current sequencing platform was significantly improved compared to the past. One aspect to realize this increased throughput is multiplexing, which may be achieved by adding a specific sequence called an adapter including an index base sequence to each DNA fragment in a process of preparing a DNA library for NGS. As a result, it is possible to pool and sequence a plurality of libraries during single sequencing. Since sequencing reads of the pooled library need to be identified and aligned by a process called demultiplexing before final data analysis, there is a disadvantage in that an element of complexity is added to increase the processing speed due to multiplexing.

In particular, the adapter may cause errors in nucleic acid sequencing results due to errors caused by polymerase during a polymerase chain reaction and/or errors in detection during the nucleic acid sequencing process, but these errors have a problem in hindering detection of mutations (Kircher et al., 2012, Nucleic Acids Res., Vol. 40, No. 1).

Meanwhile, the adapters used in the NGS are an essential element required in the process of constructing the DNA library, but when these adapters are not ligated to a DNA insert, but remain in a free state, in a subsequent experimental process, index hopping and unique molecular identifier (UMI) mix (a phenomenon in which a read with one UMI is changed to reads with different UMIs by PCR), and the like are caused to reduce the reliability of the results.

Accordingly, there is an increasing demand for enhancing the reliability of sequencing by efficiently removing a free adapter generated during the process of constructing the DNA library for NGS.

### DISCLOSURE

### TECHNICAL PROBLEM

Therefore, the present inventors have repeated many researches to solve the problem of reduced reliability of sequencing due to free adapters present in a DNA library for NGS, and as a result, found that the free adapters may be very efficiently removed by using a double-stranded nucleic acid molecule comprising a sense strand containing a type IIs restriction enzyme recognition site and an anti-sense strand of a complementary sequence thereof, and then completed the present invention.

An object of the present invention is to provide a double-stranded nucleic acid molecule comprising a sense strand containing a type IIs restriction enzyme recognition site and a random nucleotide sequence of a length of 3 to 30 nucleotides (nt); and an anti-sense strand comprising a complementary sequence to the sense strand and a 3'-end A tail.

Another object of the present invention is to provide a composition comprising the double-stranded nucleic acid molecule, DNA ligase and a type IIs restriction enzyme.

Another object of the present invention is to provide a kit for removing free adapters in an indexed DNA library for next generation sequencing, comprising the double-stranded nucleic acid molecule, DNA ligase and a type IIs restriction enzyme.

Another object of the present invention is to provide a method for removing free adapters in a DNA library for next generation sequencing, comprising steps of (a) treating and reacting a double-stranded nucleic acid molecule according to the present invention and DNA ligase in a DNA library for next generation sequencing constructed by linking the adapters for next generation sequencing to both ends of a double-stranded DNA fragment of a genomic DNA to be analyzed, and (b) treating the resulting reaction products with a type IIs restriction enzyme.

Another object of the present invention is to provide uses of the double-stranded nucleic acid molecule, DNA ligase and a type IIs restriction enzyme for preparing an agent for removing free adapters in an indexed DNA library for next generation sequencing.

### TECHNICAL SOLUTION

In order to achieve the object of the present invention, the present invention provides a double-stranded nucleic acid molecule comprising a sense strand containing a type IIs restriction enzyme recognition site and a random nucleotide sequence of a length of 3 to 30 nucleotides (nt); and an anti-sense strand comprising a complementary sequence with the sense strand and a 3'-end A tail.

In order to achieve another object of the present invention, the present invention provides a composition comprising the double-stranded nucleic acid molecule, DNA ligase and a type IIs restriction enzyme.

In order to achieve another object of the present invention, the present invention provides a kit for removing free adapters in an indexed DNA library for next generation sequencing, comprising the double-stranded nucleic acid molecule, DNA ligase and a type IIs restriction enzyme.

In order to achieve another object of the present invention, the present invention provides a method for removing free adapters in a DNA library for next generation sequencing, comprising steps of (a) treating and reacting a double-stranded nucleic acid molecule according to the present invention and DNA ligase in a DNA library for next generation sequencing constructed by linking the adapters for next generation sequencing to both ends of a double-stranded DNA fragment of a genomic DNA to be analyzed, and (b) treating the resulting reaction products with a type IIs restriction enzyme.

In order to achieve another object of the present invention, the present invention provides uses of the double-stranded nucleic acid molecule, DNA ligase and a type IIs restriction enzyme for preparing an agent for removing free adapters in an indexed DNA library for next generation sequencing.

Hereinafter, the present invention will be described in detail.

In the present invention, unless otherwise specified, a base sequence is disclosed in a 5' to 3' direction.

In the present invention, the term 'strand' is used interchangeably with 'strand', and refers to a plurality of nucleotide polymers (i.e., polynucleotide).

The present invention provides a double-stranded nucleic acid molecule comprising a sense strand containing a type IIs restriction enzyme recognition site and a random nucleotide sequence of a length of 3 to 30 nucleotides (nt); and an anti-sense strand containing a complementary sequence to the sense strand and a 3'-end A tail.

In the double-stranded nucleic acid molecules provided by the present invention, free adapters that are not ligated to a insert may be ligated to a 5'-end of the sense strand and a 3'-end of the anti-sense strand when constructing a library for next generation sequencing, and thus may be used as a platform for cutting the free adapters through the type IIs restriction enzyme.

This effect of the double-stranded nucleic acid molecules provided by the present invention may be confirmed in more detail through Examples of the present invention.

In the present invention, the term 'restriction enzyme (restriction endonuclease)' is an enzyme that selectively cuts phosphodiester bonds between nucleic acids in double-stranded DNA to make fragments. All restriction enzymes recognize a specific base sequence of DNA, and this base sequence indicates a selective action site of the restriction enzyme. The restriction enzyme recognizes a specific base sequence at a recognition site and cuts a nucleic acid at a restriction site (cutting site).

In the present invention, the type IIs restriction enzyme refers to an enzyme which recognizes an asymmetric DNA sequence and in a specific group cut outside the restriction enzyme recognition site, generally in 1 to 25 nucleotides from the restriction enzyme recognition site. This type of enzyme differs from other restriction enzymes in that the recognition sequence is separated from the cutting site. In the present invention, some examples of the type IIs restriction enzyme include MmeI, FokI, Alw26I, BbvI, BsrI, Earl, HphI, MboI, SfaNI, AlwI, BsaI, BbsI, BbuI, BsmAI, BsmI, BspMI, Esp3I, HgaI, MboII, PleI, SfaNi, Mnll, CspCI, AloI, PpiI, PsrI, BplI, Fall, Bsp24I, BsaXI, HaeIV, CjeI, CjePI, Hin4I, BaeI, AlfI, BcgI, BslFI, Tth111I, and the like, but are not limited thereto. Many of the type IIs restriction enzymes are commercially available and recognition sites thereof are well known to those skilled in the art. The recognition site of the restriction enzyme generally consists of 4 to 8 bases, and the specific sequence thereof may be easily confirmed by those skilled in the art depending on a type of restriction enzyme to be selected.

In the present invention, non-limiting examples of the type IIs restriction enzyme, sequences of theses restriction enzyme recognition sites, and the positions of restriction sites were shown in Table 1 below.

**[Table 1]**

| Type IIs | Restriction enzyme recognition sequence and distance from restriction enzyme recognition sequence to cutting position (sense/anti-sense) |
|---|---|
| MmEI | TCCRAC(20/18) (SEQ ID NO: 4) |
| FokI | GGATG(9/13) (SEQ ID NO: 5) |
| BbvI | GACGC(5/10) (SEQ ID NO: 6) |
| HgaI | GACGC(5/10) (SEQ ID NO: 7) |
| CspCI | (11/13)CAA(N)₅GTGG(12/10) (SEQ ID NO: 8) |
| AloI | (7/12)GAAC(N)₆TCC(12/7) (SEQ ID NO: 9) |
| PpiI | (7/12)GAA(N)₅CTC(13/8) (SEQ ID NO: 10) |
| PsrI | (7/12)GAAC(N)₆CTC(13/8) (SEQ ID NO: 11) |
| BplI | (8/13)GAG(N)₅CTC(13/8) (SEQ ID NO: 12) |
| Fall | (8/13)AAG(N)₅CTT(13/8) (SEQ ID NO: 13) |
| Bsp24I | (8/13)GAC(N)₆TGG(12/7) (SEQ ID NO: 14) |
| BsaXI | (9/12)AC(N)₅CTCC(10/7) (SEQ ID NO: 15) |
| HaeIV | (7/13)GAY(N)₅RTC(14/9) (SEQ ID NO: 16) |
| CjeI | (8/14)CCA(N)₆GT (SEQ ID NO: 17) |
| CjePI | (7/13)CCA(N)₇TC(14/8) (SEQ ID NO: 18) |

According to an exemplary embodiment, in the present invention, the type IIs restriction enzyme may be MmeI, and the restriction enzyme recognition site sequence thereof may consist of TCCRAC (sense strand) (SEQ ID NO: 4) and 3'-AGGYTG-5' (anti-sense strand) (SEQ ID NO: 19).

Meanwhile, the sense strand of the double-stranded nucleic acid molecule of the present invention includes a random nucleotide sequence of a length of 3 to 30 nucleotides (nt) bound to a 3'-end of the restriction enzyme recognition site sequence. The random nucleotide sequence facilitates annealing of the sense strand and the anti-sense strand, and may act as a structure in which the type IIs restriction enzyme may act. The nucleotide sequence and length of the random nucleotide facilitate annealing of the sense strand and the anti-sense strand, and is not particularly limited as long as the random nucleotide acts as a structure in which the restriction enzyme may act. The random nucleotide may be easily set in consideration of the nucleotide sequence and length of the restriction enzyme recognition site.

Preferably, the random nucleotide sequence bound to the 3'-end of the sense strand of the restriction enzyme recognition site may be configured so that a melting temperature (Tm) value of the sense strand and the anti-sense strand of the double-stranded nucleic acid molecule may be 20°C or higher. Specifically, the Tm value of the double-stranded nucleic acid molecule may include any range in a range having a lower limit and an upper limit of any two values selected from the group consisting of 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C and 70°C, or any specific value of the temperatures listed above.

More preferably, the random nucleotide sequence bound to the 3'-end of the sense strand of the restriction enzyme recognition site may be configured so that a melting temperature (Tm) value of the sense strand and the anti-sense strand of the double-stranded nucleic acid molecule may be higher than 37°C. Specifically, the Tm value of the double-stranded nucleic acid molecule may include any range in a range having a lower limit and an upper limit of any two values selected from the group consisting of 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C and 70°C, or any specific value of the temperatures listed above.

When the Tm value is lower than the temperatures, the double-stranded nucleic acid molecules are denatured into single-stranded nucleic acid molecules under conditions of treating a restriction enzyme, and thus, there may be a problem that a normal function is not exhibited.

As used herein, the term "melting temperature (Tm)" refers to a melting temperature at which half of double-stranded nucleic acid molecules become single-stranded.

In addition, the random nucleotide sequence bound to the 3'-end of the restriction enzyme recognition site sequence may have a length of 3 to 30 nt, and includes any range in a range having a lower limit and an upper limit of any two values selected from the group consisting of 3, 4, 5, 6, 7, 8, 9, 10, 11, 12, 13, 14, 15, 16, 17, 18, 19, 20, 21, 22, 23, 24, 25, 26, 27, 28, 29 and 30 nt, or any specific value of the temperatures listed above. The random nucleotide sequence may have a length of preferably 4 to 28 nt, more preferably 5 to 25 nt, even more preferably 6 to 20 nt, and most preferably 6 to 10 nt.

When the length of the random nucleotide sequence bound to the 3'-end of the restriction enzyme recognition site sequence is too short, the Tm value of the double-stranded nucleic acid molecule is lowered, so that the double-stranded nucleic acid molecule is denaturated into single-stranded nucleic acid molecules under a restriction enzyme treatment condition or may not function as a structure in which the restriction enzyme may act. On the contrary, when the length of the random nucleotide sequence bound to the 3'-end of the restriction enzyme recognition site sequence is too long, it is not preferred in an economic aspect, and it is not preferred that the sense and/or anti-sense strands of the double-stranded nucleic acid molecule do not form a restriction enzyme recognition site and are more likely to form an abnormal secondary structure.

According to an exemplary embodiment, the random nucleotide sequence may be TTGTGC (sense strand) (SEQ ID NO: 20) and 3'-CCACAA-5' (anti-sense strand) (SEQ ID NO: 21).

In the present invention, the anti-sense strand may include a complementary sequence to the sense strand and a 3'-end A tail.

In the present invention, the term 'complementary' refers to a sequence-specific binding relationship in which adenine (A) binds to thymine (T) or uracil (U) and guanine (G) binds to cytosine (C), when being used with respect to a nucleic acid. For example, a nucleic acid having a sequence GCAU (SEQ ID NO: 22) in a 5' to 3' direction is complementary to a nucleic acid having a sequence CGTA (SEQ ID NO: 23) in a 5' to 3' direction. In the present disclosure, the term 'complementary' is used to encompass both substantially complementary nucleic acid and 100% complementary nucleic acid (i.e., does not allow mismatch), and most preferably, a relationship of 100% complementary nucleic acid.

The adenine tail (A-tail) refers to one adenine nucleotide bound to the 3'-end of the anti-sense strand, and may inhibit the generation of concatemers during ligation between the double-stranded nucleic acid molecules of the present invention and adapters used for constructing the DNA library, and enables ligation with complementary dT overhangs present in the adapters.

In one aspect of the present invention, a random nucleotide sequence of a length of 1 to 10 nt may be further included at the 3'-end of the sense strand or the 5'-end of the anti-sense strand. Specifically, a random nucleotide sequence of a length of 1 nt, 2 nt, 3 nt, 4 nt, 5 nt, 6 nt, 7 nt, 8 nt, 9 nt or 10 nt may be further contained in the 3'-end of the sense strand or the 5'-end of the anti-sense strand. Preferably, a random nucleotide sequence of a length of 2 nt, 3 nt, 4 nt, 5 nt, 6 nt, or 7 nt may be further contained in the 3'-end of the sense strand or the 5'-end of the anti-sense strand. More preferably, a random nucleotide sequence of a length of 2 nt, 3 nt, 4 nt, or 5 nt may be further contained in the 3'-end of the sense strand or the 5'-end of the anti-sense strand. Most preferably, a random nucleotide sequence of a length of 2 nt or 3 nt may be further contained in the 3'-end of the sense strand or the 5'-end of the anti-sense strand.

In one aspect of the present invention, the random nucleotide sequence further contained in the 3'-end of the sense strand may consist of only cytosine (C), guanine (G), or a combination thereof, and the base sequence of the random nucleotide further contained in the 5'-end of the anti-sense strand may consist of only thymine (T), cytosine (C), guanine (G) or a combination thereof. For example, the random nucleotide sequence further contained in the 3'-end of the sense strand may be C, G, CC, GG, CG, GC, CCC, GGG, CCG, GGC, CGC, GCG, and the like, and the random nucleotide sequence further contained in the 5'-end of the anti-sense strand may be C, T, G, TT, TC, CT, TG, GT, CC, GG, CG, GC, TTT, TTC, TCT, CTC, CCT, TCC, CCT, TTG, TGT, GTG, GGT, TGG, CCC, GGG, CCG, GGC, CGC, GCG, and the like, but the present invention is not limited thereto.

The random sequence of a length of 1 to 10 nt further contained in the 3'-end of the sense strand or the 5'-end of the anti-sense strand may prevent free adapters which are not ligated to the insert when constructing the library for next generation sequencing from being non-specifically ligated to an opposite end of the restriction enzyme recognition site in the double-stranded nucleic acid molecules.

In another aspect of the present invention, in the double-stranded nucleic acid molecules of the present invention, the 3'-end of the sense strand and the 5'-end of the anti-sense strand are connected by a loop to have a hairpin structure.

As used herein, the term 'hairpin' refers to a self-hybridized structure that takes a stem-loop structure when two complementary regions in a single chain are separated by a non-complementary region to form a double strand. In the two complementary regions (the sense strand and the anti-sense strand in the present invention), if the complementary degree and the orientation of the complementary region are sufficient, sufficient base pairing occurs to form a double structure called a stem, and a non-complementary region forms a loop structure and connects the two complementary regions. The loop region is formed by the absence of base pairing between nucleic acids (or nucleic acid analogs) of the loop region, and the loop region of the hairpin structure is a single-stranded region between the sense and anti-sense strands, which may be referred to as an 'intervening single strand'.

In this specification, the two complementary regions are referred to as two arms, and thus, in this specification, the stem is defined as consisting of two arms (3'arm and 5'arm). Therefore, the hairpin structure may mean a double structure formed while a 3' arm (i.e., a 3' end of a single chain) and a 5' arm (i.e., a 5' end of a single chain) having a complementary relationship in the single chain are separated from each other by a non-complementary loop sequence.

When the double-stranded nucleic acid molecule of the present invention is separated by the non-complementary loop sequence to from a hairpin-shaped double structure, the loop may consist of 4 to 80, 4 to 75, 4 to 70, 4 to 65, 4 to 60, 4 to 55, 4 to 50, 4 to 45, 4 to 40, 4 to 35, 4 to 30, 4 to 25, 4 to 20, 4 to 15 or 4 to 10 bases, and in the exemplary embodiment of the present invention, the loop may consist of 6 nucleotides. In addition, when some base sequences constituting the loop are complementary to each other, Watson-Crick base pairing may be formed to have a stem structure. The stem structure may include an AU motif in which Watson-Crick base pairing is formed between A and U.

In an exemplary embodiment according to the present invention, a sequence of 6 bases constituting the loop is AAAAAA (SEQ ID NO: 24).

When the double-stranded nucleic acid molecule of the present invention is separated by the non-complementary loop sequence to have a hairpin-shaped double structure, the nucleic acid molecule may have a structure of 5'- type IIs restriction enzyme recognition site - random nucleotide sequence of a length of 3 to 30 nucleotides (nt) - hairpin loop - complementary sequence to the random nucleotide sequence of the length of 3 to 30 nucleotides (nt) - complementary sequence to the type IIs restriction enzyme recognition site - A tail -3'.

In the double-stranded nucleic acid molecule of the hairpin structure, the random nucleotide sequence of the length of 3 to 30 nt may be referred to as described above.

Since the double-stranded nucleic acid molecule of the hairpin structure forms a hairpin structure at an opposite end of the restriction enzyme recognition site, it is possible to prevent free adapters that are not ligated to the insert when constructing the library for next generation sequencing from being non-specifically ligated.

According to an exemplary embodiment of the present invention, the double-stranded nucleic acid molecule of the present invention may be a double-stranded nucleic acid molecule in which a sense strand of a base sequence defined by SEQ ID NO: 1 and an anti-sense strand of a nucleotide sequence defined by SEQ ID NO: 2 are complementarily bound to each other, or may be a nucleic acid molecule consisting of a nucleotide sequence defined by SEQ ID NO: 3, which is an intervening single strand separated by the non-complementary loop sequence.

The present invention provides a composition comprising the double-stranded nucleic acid molecules described above, DNA ligase and a type IIs restriction enzyme.

In the present invention, the composition may be used for improving sample detection ability in an indexed DNA library in next generation sequencing (NGS) or for removing free adapters that are not ligated to the insert in the process of constructing the indexed DNA library of next generation sequencing.

The next generation sequencing may be largely divided into two steps. The first step is a step of constructing a DNA library that makes DNA to be analyzed in a form that can be sequenced by a next generation sequencing device, and the second step is a step of analyzing the constructed DNA library by the next generation sequencing device.

In general, the step of constructing the 'DNA library' in the present invention includes the following steps. For convenience, a next generation sequencing device from Illumina Co., Ltd. will be described as an example. In the present specification, for convenience of description, only the sequencing device from Illumina Co., Ltd is exemplified, and other general next generation sequencing devices may be used without limitation.
1) A genomic DNA to be analyzed is fragmented to a predetermined length by sonication or enzymatic DNA digestion. However, when the DNA is fragmented, such as cell-free DNA, the next step proceeds without such decomposition.
2) End Repair: The lengths of both ends of two single-stranded DNAs constituting the fragmented double-stranded DNA are the same as each other.
3) dA-Tailing: One base A is linked to the 3' end of the fragmented double-stranded DNA.
4) Adapter linkage: For sequencing by the next generation sequencing device, an adapter including an index or UMI is linked to the double-stranded DNA fragment.
5) Polymerase Chain Reaction: The DNA to be analyzed is amplified through PCR.

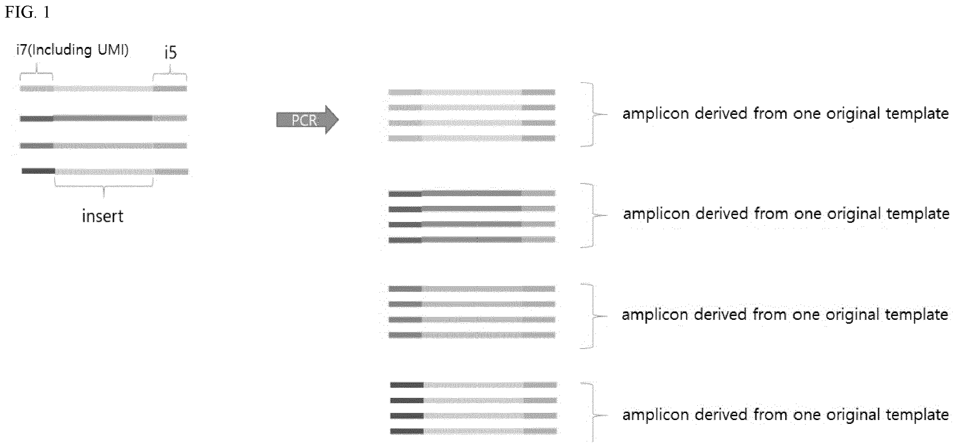

A DNA molecule pool generated through the process is a general 'DNA library' in the art, and the term 'DNA library' used in the present invention may also be interpreted in the same meaning. In the present invention, the 'indexed DNA library' means that a adapter including an index sequence is ligated to one or both ends of the double-stranded DNA fragment to be analyzed (see FIG. 1).

In the present invention, the 'adapter' may include a sequencing primer site, which binds to a sequencing primer, a unique sequence suitable for use in sequencing (e.g., NGS), a sample index sequence, and a flow cell binding sequence.

In addition, in the present invention, the adapter may further include a randomized DNA barcode or a unique molecular identifier (UMI) between a primer site (that is, a site to which the sequencing primer binds) which is a unique sequence suitable for use in sequencing (e.g., NGS) and a sample index sequence or between the sample index sequence and a flow cell binding sequence.

The randomized DNA barcode may refer to, for example, SHAPE-SEQ, Document [Lucks et al., Proc Natl Acad Sci US A 108: 11063-11068], and the UMI may be understood by referring to Documents [Kivioja et al., Nature Methods 9, 72-74 (2012)]; [Islam et al., Nature Methods 11, 163-166 (2014)]; and [Karlsson et al., Genomics. 2015 Mar;105(3):150-8].

The sequence suitable for use in sequencing may be selected to be used with a desired sequencing method, for example, a next generation sequencing method, for example, a library construction method such as Illumina, Ion Torrent or Roche/454; an lllumina Solexa Genome Analyzer, an Applied Biosystems) SOLiD^{™} system, an Ion Torrent^{™} semiconductor sequencer, PacBio^{®} real-time sequencing and Helicos^{™} single molecule sequencing (SMS). For example, the sequence suitable for use in sequencing may refer to WO2014020137, and Documents [Voelkerding et al., Clinical Chemistry 55:4 641-658 (2009)] and [Metzker, Nature Reviews Genetics 11:31-46 (2010)]. A ThruPLEX DNA-seq kit (Rubicon; see US Pat. Nos. 7,803,550; 8,071,312; 8,399,199; and 8,728,737) and many kits including NEBNext^{®} (New England BioLabs; e.g., see US Pat. 8,420,319) are commercially available for the construction of the DNA library for NGS.

The DNA library generated through the PCR includes an index sequence, or both an index sequence and a randomized DNA barcode sequence (or UMI), wherein the index sequence serves to classify a sample to which the genomic DNA to be analyzed belongs during next generation sequencing, and the randomized DNA barcode sequence (or UMI) may serve to assign a barcode to a single-stranded DNA fragment of the genomic DNA to be analyzed.

In the construction of the DNA library for next generation sequencing, the linking of the adapter for next generation sequencing to a double-stranded DNA fragment of the genomic DNA to be analyzed is a required step for sequencing. The method for constructing the DNA library using the adapter including the index sequence has an advantage of performing multiplex sequencing by enabling differentiation between different samples.

However, during the process of constructing the DNA library, a free adapter that is not ligated with a target DNA fragment (or insert) causes index hopping or UMI mix, thereby reducing the reliability of the NGS results.

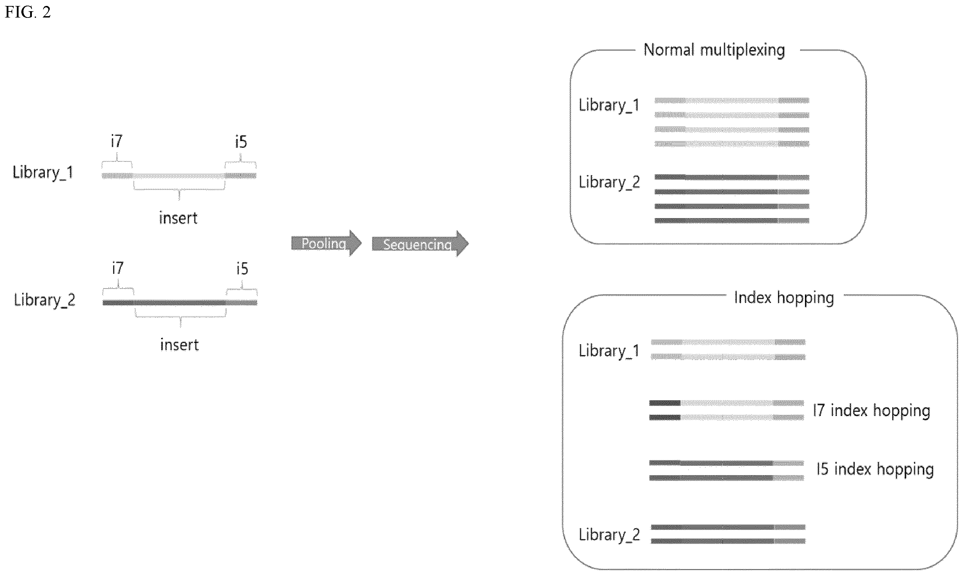

The 'index hopping' means that when sequencing is performed in multiplex after mixing DNA libraries from each sample including different indexes, in the PCR process, a primer including a free adapter or an index including a specific index sequence derived from each sample is not removed, but functions as a primer in a DNA library having different indexes so that DNA library information (that is, index information) is mixed (see FIG. 2).

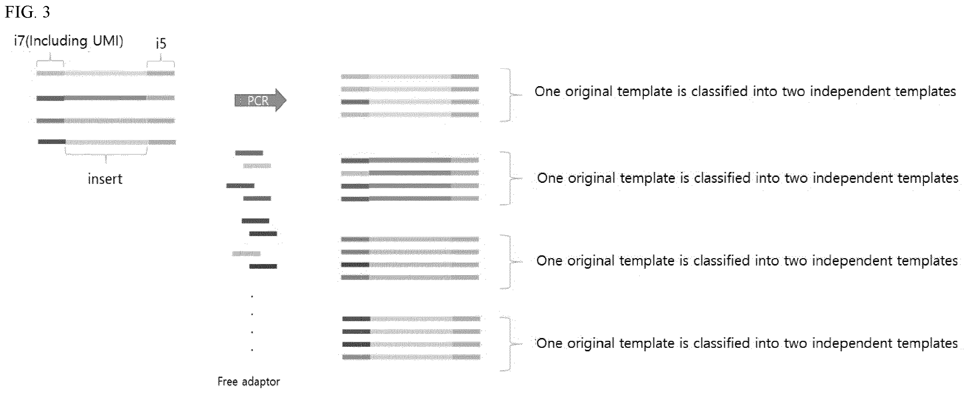

The 'UMI mix' means that the UMI labeling a specific fragment is mixed in the same sample to cause a bias, and occurs when the UMI of the free adapter is introduced through the PCR process while the free adapter functions as the primer (see FIG. 3).

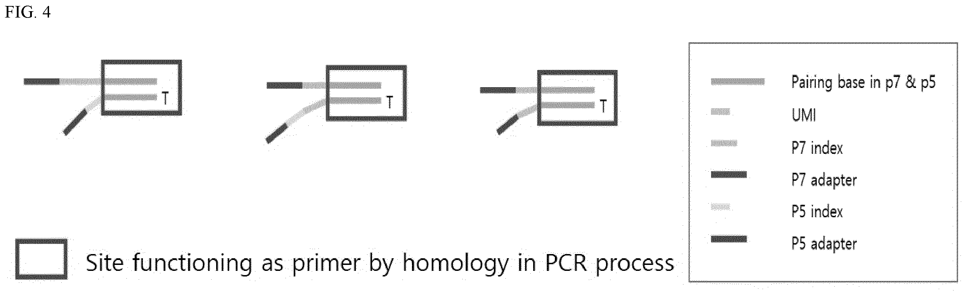

An example of a region in the free adapter functioning as the primer in the PCR process is illustrated in FIG. 4.

The composition of the present invention may be used for cutting the free adapter which is not bound to the target DNA fragment in the process of constructing the indexed DNA library to significantly lower the possibility of index hopping and/or UMI mix, thereby improving the detection ability of the NGS results.

More specifically, the composition of the present invention may cut a part or all of a sequencing primer binding site, which is a region with homology capable of functioning as a primer in a free adapter to separate the sequencing primer binding site and the UMI (or barcode) sequence and/or the index sequence, thereby significantly lowering the possibility of index hopping and/or UMI mix.

In an aspect of the present invention, when the double-stranded nucleic acid molecules and the DNA ligase included in the composition of the present invention are added to a DNA library suspected of including the free adapter, the adapter binds to the double-stranded nucleic acid molecule of the present invention by the action of DNA ligase in the same manner as binding to DNA fragments to form an adapter-double-stranded nucleic acid molecule complex. In the complex, since a type IIs restriction enzyme recognition site constituting the double-stranded nucleic acid molecule is included, and a type IIs restriction enzyme has a cutting site in 1 to 20 nucleotides from the recognition site, in the adapter-double-stranded nucleic acid molecule complex, the sequence in the adapter region is cut by treatment with the type IIs restriction enzyme.

A process of excluding index hopping and/or UMI mix by cutting the free adapter by the composition provided by the present invention is sequentially described as follows:
(i) One end of a free adapter, that is, an index or UMI is included in one end of the double-stranded nucleic acid molecule provided by the present invention, i.e., the 5' end of the sense strand and the 3' end of the anti-sense strand, and the end site of the adapter including the double stranded structure for ligation is ligated.
(ii) A restriction enzyme recognizes a type IIs restriction enzyme recognition site included in the double-stranded nucleic acid molecule provided by the present invention.
(iii) A sequence in the free adapter is cut according to a characteristic of a type IIs restriction enzyme that cuts a sequence in 1 to 25 nucleotides from the restriction enzyme recognition site.

The part cut by the restriction enzyme in (iii) may be a region in a sequencing primer binding site of the free adapter, a boundary region between the sequencing primer binding site and a UMI (or barcode) sequence, a boundary region between the sequencing primer binding site and an index sequence, a region in the UMI (or barcode) sequence, or a region in the index sequence.

The cut adapter may then be washed with a buffer or completely removed from the DNA library by bead clean-up.

In an aspect of the present invention, the sequence of the restriction enzyme recognition site and the type of the type IIs restriction enzyme in the double-stranded nucleic acid molecule included in the composition may be appropriately selected by those skilled in the art according to the length and sequence of the sequencing primer binding site in the adapter used for constructing the DNA library.

It is most preferred to use a double-stranded nucleic acid molecule comprising a type IIs restriction enzyme and a recognition site sequence thereof exhibiting a restriction site (i.e., a cutting site) that is longer than or equal to the length of the sequencing primer binding site in the adapter. Even if using a double-stranded nucleic acid molecule including a type IIs restriction enzyme and a recognition site sequence thereof representing a restriction site shorter than the length of the sequencing primer binding site in the adapter, if the Tm values represented by the sequence of the sequencing primer binding site remaining in the UMI (or barcode) sequence and/or the index sequence after cutting and the complementary sequence thereof are significantly lower than the annealing temperature in the PCR process, the possibility of the problem of index hopping or UMI mix is very low.

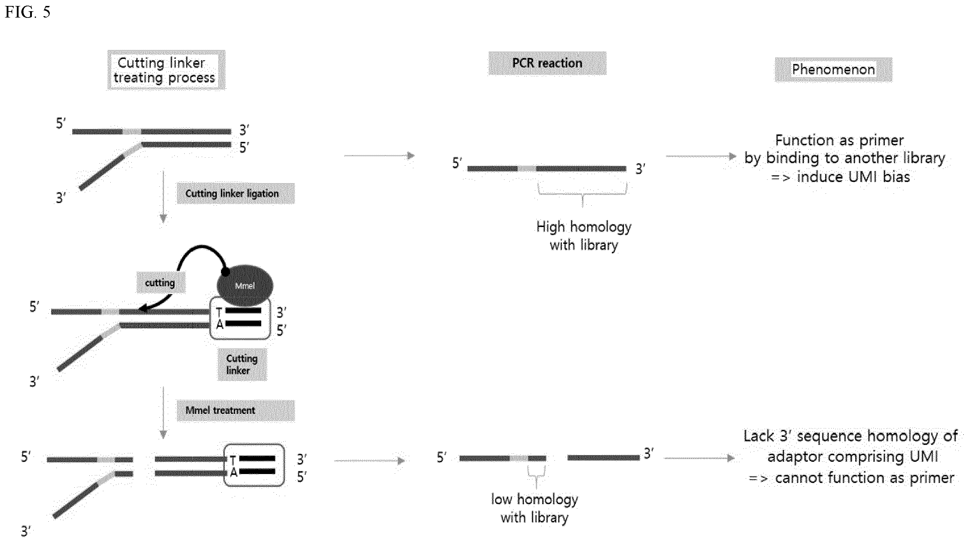

An operation method of double-stranded nucleic acid molecules according to the present invention is illustrated in FIG. 5.

In the present invention, the 'DNA ligase' refers to an enzyme that catalyzes bonds between nucleotides by forming a phosphodiester bond according to decomposition of nucleotide triphosphate such as ATP and the like. The type of the DNA ligase is not particularly limited so long as the double-stranded nucleic acid molecules of the present invention may be ligated, and non-limiting examples thereof may be T4 DNA ligase, T3 DNA ligase, T7 DNA ligase, E. coli DNA ligase, Ampligase DNA ligase, CircLigase^{™} ssDNA ligase, or a combination thereof, preferably T4 DNA ligase, but are not limited thereto.

The composition of the present invention may further include a reagent required for ligation and restriction enzyme reaction, such as a buffer, a cofactor (e.g., Mg²⁺), ATP, and the like without limitation, in addition to the double-stranded nucleic acid molecule, the DNA ligase and the type IIs restriction enzyme described above.

The present invention provides a kit for removing free adapters in an indexed DNA library for next generation sequencing, comprising the double-stranded nucleic acid molecules, the DNA ligase and the type IIs restriction enzyme described above.

The present invention provides a method for removing free adapters in a DNA library for next generation sequencing, comprising steps of (a) treating and reacting a double-stranded nucleic acid molecule according to the present invention and DNA ligase in a DNA library for next generation sequencing constructed by linking the adapters for next generation sequencing to both ends of a double-stranded DNA fragment of a genomic DNA to be analyzed, and (b) treating the resulting reaction products with a type IIs restriction enzyme.

The method of the present invention may further include washing with a buffer or performing bead clean-up after step (b).

Further, the present invention provides uses of the double-stranded nucleic acid molecule, the DNA ligase and the type IIs restriction enzyme for preparing an agent for removing free adapters in an indexed DNA library for next generation sequencing.

### ADVANTAGEOUS EFFECTS

According to the double-stranded nucleic acid molecules, the composition, and the method provided by the present invention, only the free adapters in the indexed DNA library for next generation sequencing may be selectively removed to prevent problems of index hopping, UMI mix, and the like caused by the free adapters during the next generation sequencing.

### DESCRIPTION OF DRAWINGS

FIG. 1 is a diagram schematically illustrating the definition of an indexed DNA library.
FIG. 2 is a diagram schematically illustrating a concept of index hopping.
FIG. 3 is a diagram schematically illustrating a concept of UMI mix.
FIG. 4 illustrates four regions of a free adapter that may act as a primer in a PCR process during a process of constructing a DNA library.
FIG. 5 is a diagram schematically illustrating an operation method of a double-stranded nucleic acid molecule according to the present invention.
FIGS. 6A and 6B are diagrams schematically illustrating examples of the double-stranded nucleic acid molecule according to the present invention.
FIG. 7 is a diagram for confirming a result without performing bead clean-up in order to confirm whether an adapter is fragmented by treatment with a restriction enzyme MmeI.
FIG. 8 is a diagram schematically illustrating which region each band represents in the adapter in the result of FIG. 7.
FIG. 9 is a result of confirming whether non-specific decomposition appears after treatment with MmeI to a PCR product in order to confirm whether non-specific cutting occurs by treatment with a restriction enzyme.
FIG. 10 is a diagram illustrating an experiment method for confirming how much a UMI bias is caused by free adapters in an NGS sequencing process of a sample DNA and whether such a UMI bias may be removed by the double-stranded nucleic acid molecule according to the present invention and the restriction enzyme.
FIG. 11 is a diagram illustrating an experiment method for confirming how much a UMI bias is caused by free adapters in an NGS sequencing process of a sample DNA and whether such a UMI bias may be removed by the double-stranded nucleic acid molecule according to the present invention and the restriction enzyme.

### MODES FOR THE INVENTION

Hereinafter, the present invention will be described in detail by the following Examples. However, the following Examples are just illustrative of the present invention, and the contents of the present invention are not limited to the following Examples.

### Experiment method

### 1. Preparation of double-stranded nucleic acid molecule and nucleic acid molecule having hairpin structure

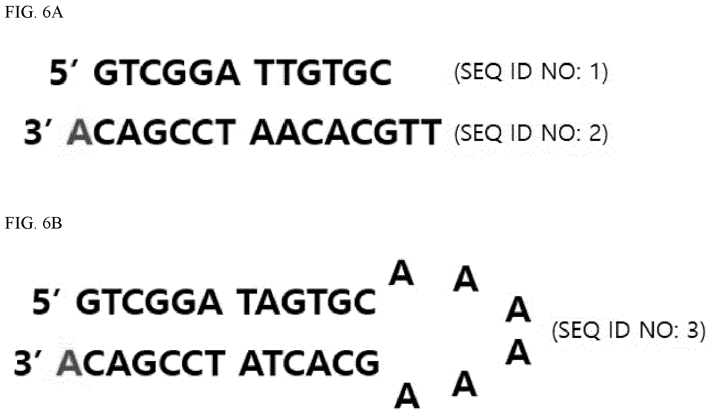

A sense strand of SEQ ID NO: 1 in which a random base sequence TTGTGC (SEQ ID NO: 20) providing a structure in which a restriction enzyme may operate was bound to a 3' end of a recognition site sequence GTCGGA (SEQ ID NO: 25) of a type IIs restriction enzyme Mmel and an anti-sense strand of SEQ ID NO: 2 in which TT was additionally bound to a 5' end of a base sequence complementary to the sense strand and A was additionally added to the 3' end were prepared. The sense strand and the anti-sense strand were mixed with the same moles, denatured at 50°C, and then cooled down slowly at room temperature to form a double-stranded nucleic acid molecule. The double-stranded nucleic acid molecule was referred to as cutting linker_FR (FIG. 6A).

In addition, a single-stranded nucleic acid molecule consisting of a base sequence of SEQ ID NO: 3 was prepared, denatured at 50°C, and then cooled down slowly, and induced to form an intra structure to form a nucleic acid molecule having a hairpin structure. The nucleic acid molecule having the hairpin structure was referred to as cutting linker_hairpin (FIG. 6B).

### 2. Ligation of free adapter and double-stranded nucleic acid molecule

The adapter was treated with the prepared double-stranded nucleic acid molecule at a ratio of 1:10, and was treated with T4 DNA ligase to induce ligation thereof.

Specifically, experimental materials listed in Table 2 below were mixed and incubated at 25°C for 2 hours, and then heat-inactivated at 65°C for 20 minutes to terminate the reaction.

**[Table 2]**

| **Reagent** | **Volume (ul)** | **Product information** |
|---|---|---|
| Adaptor (3 uM) | 2.5 | IDT order production |
| Cutting linker FR or Cutting linker hairpin | 25 | IDT order production |
| T4 ligase | 1 | NEB (Cat.M0202) |
| T4 ligase buffer (10X) | 4 | |
| TDW | 7.5 | |
| Total | 40 | |

### 3. Treatment of restriction enzyme

A reaction mixture reacted according to Experimental Method 2 was treated with a type IIs restriction enzyme, MmeI to induce a restriction enzyme reaction.

Specifically, experimental materials listed in Table 3 below were mixed and incubated at 37°C for 1 hour, and then heat-inactivated at 65°C for 20 minutes to terminate the reaction.

**[Table 3]**

| **Reagent** | **Volume (ul)** | **Product information** |
|---|---|---|
| Reaction mixture (Experimental Method 2) | 40 | |
| Mmel (2 units/ul) | 5 | NEB (Cat.R0637) |
| SAM (1.6 mM) | 1.6 | |
| Cutsmart buffer (10X) | 1 | |
| TDW | 2.4 | |
| Total | 50 | |

### 4. Bead clean-up

By using 2.0X accubead (HiAccuBead (AccuGene (Cat. ACN01.50)), clean-up was performed according to manufacturer's instructions.

### Experimental Results

### 1. Confirmation of fragmentation of free adapter by cutting linker FR or cutting linker_hairpin, and restriction enzyme

It was confirmed whether free adapters in a DNA library may be removed by the double-stranded nucleic acid molecule prepared in the experimental method, T4 DNA ligase and a type IIs restriction enzyme MmeI. Specifically, the adapter, the double-stranded nucleic acid molecule according to the present invention, the DNA ligase, and Mmel were mixed, respectively, to induce a reaction through the process described in the experimental method, and then the result was confirmed through tapestation (Agilent).

First, in order to confirm whether the adapter was fragmented by treatment of Mmel, the result was confirmed without performing bead clean-up, and 2 ng of each sample was loaded.

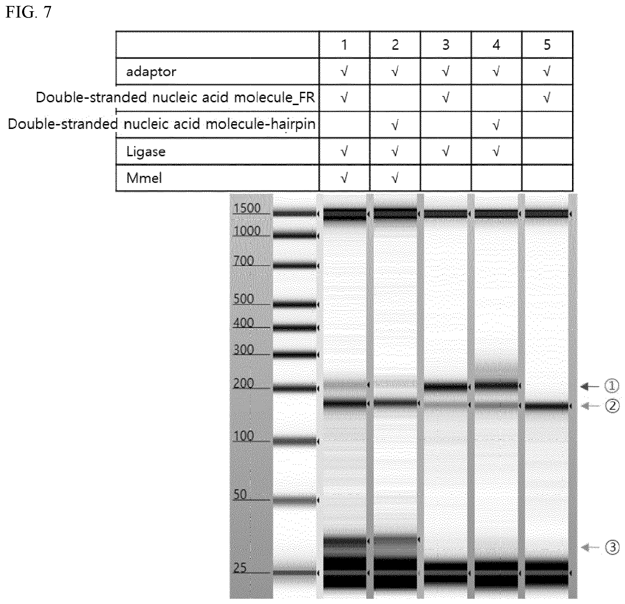

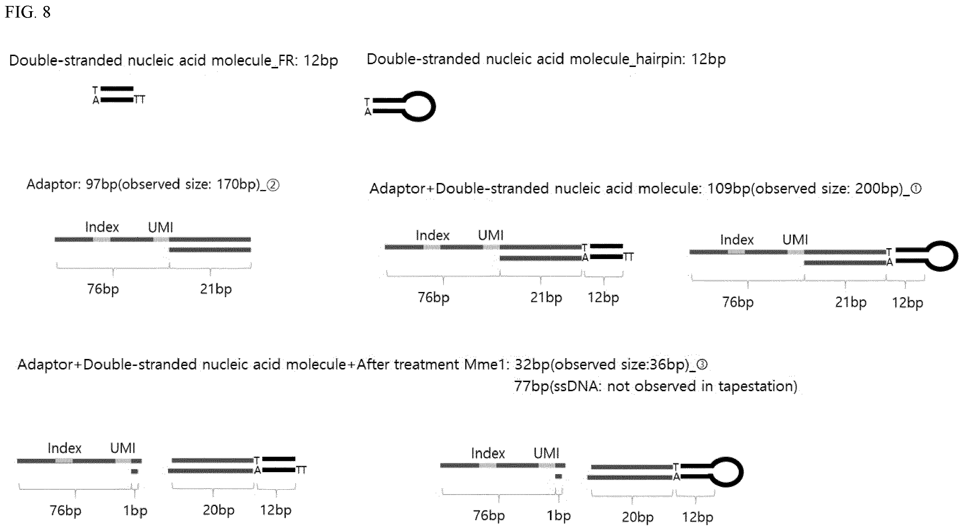

The results thereof were illustrated in FIGS. 7 and 8.

Referring to FIGS. 7 and 8, adapter fragments estimated to be cut by the double-stranded nucleic acid molecule according to the present invention and the restriction enzyme MmeI were confirmed. As shown in lanes 3 and 4 of FIG. 7, when the double-stranded nucleic acid molecule according to the present invention was ligated to the adapter, it was confirmed that a band of ② was shifted to form a band ①, and when Mmel was treated thereto, as shown in lanes 1 and 2, the band ① disappeared and a cutting fragment ③ was confirmed (see FIG. 8). A band of ③ is a product derived by cutting and fragmenting the adapter and may be removed through a clean-up process.

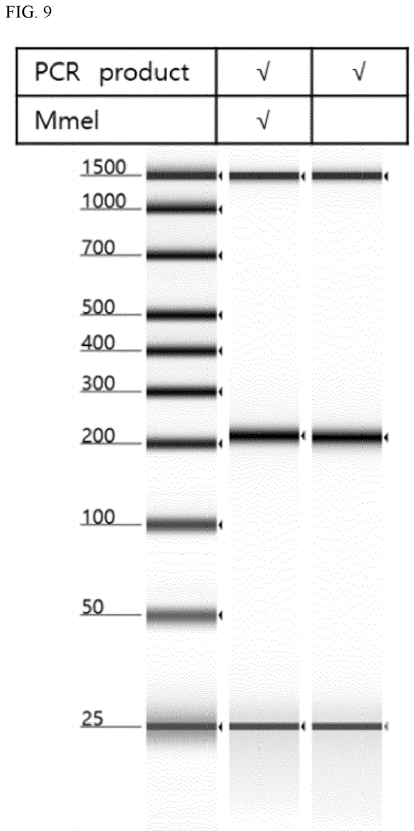

Finally, in order to confirm whether non-specific cutting by Mmel occurred, it was confirmed whether non-specific decomposition appeared after Mmel was treated in a PCR product (no Mmel recognition site). As a result, as illustrated in FIG. 9, no fragmentation was observed in no Mmel recognition site.

### 2. Removal of UMI bias through cutting of free adapter in NGS sequencing process

Through the above-described experimental results, it was confirmed that cutting linker_FR and cutting linker_hairpin provided by the present invention may efficiently cut the free adapter.

Therefore, in a subsequent experiment, how much the UMI bias was caused by the free adapter in the NGS sequencing process of the sample DNA, and whether such a UMI bias may be removed by the cutting linker FR or cutting linker hairpin and the restriction enzyme.

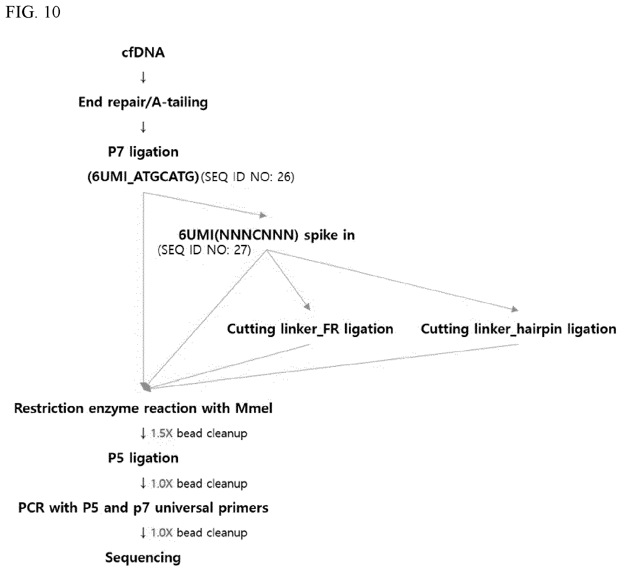

An experimental procedure was as follows (see FIG. 10):
1. It was confirmed how many UMI biases have been induced in an adapter in a process of fixing an UMI base sequence to ATGCATG (SEQ ID NO: 26) and spiking in an adapter having NNNCNNN (SEQ ID NO: 27) therein to actually construct a DNA library.
2. It was confirmed whether the UMI biases caused by the free adapter were removed by each of two types of cutting linkers (FR and hairpin).
3. For the reproducibility of the results, two independent experiments were performed, and the mean and STDEV were expressed in graphs.

In the process of constructing the DNA library, an adapter with ATGCATG (SEQ ID NO: 26) was ligated to an input DNA, and when there was no UMI bias, all DNA libraries need to have a UMI type ATGCATG (SEQ ID NO: 26). On the contrary, when there was a UMI bias, the DNA library will have a random UMI type other than ATGCATG (SEQ ID NO: 26).

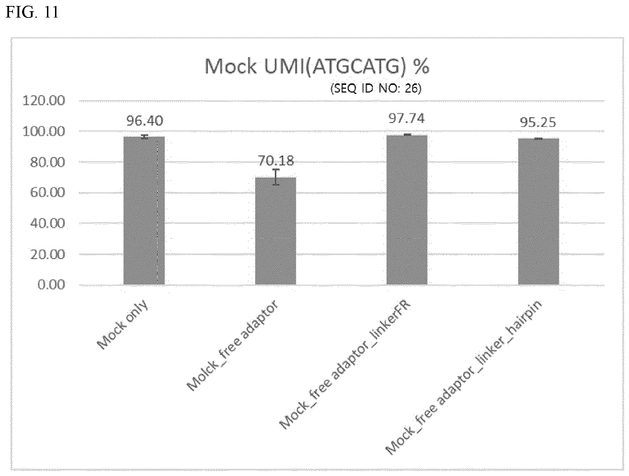

Based on this fact, % of reads with UMI of the sequence ATGCATG (SEQ ID NO: 26) in each experimental group was calculated as a result and illustrated in Table 4 below and FIG. 11.

**[Table 4]**

| | Mock only | | Mock_free adapter | | Mock_free adapter_linker FR | | Mock_free adapter_linker FR | |
|---|---|---|---|---|---|---|---|---|
| | AVER | STDEV | AVER | STDEV | AVER | STDEV | AVER | STDEV |
| Mock UMI read (%) | 96.4 | 1.07 | 70.18 | 4.95 | 97.74 | 0.08 | 95.25 | 0.28 |

In Table 4 and FIG. 11, in the case of Mock only, after sequencing a library by ligating only an adapter having a fixed UMI (ATGCATG) (SEQ ID NO: 26), % of reads with the fixed UMI among all reads was obtained. In the case of Mock free adapter, the fixed UMI was inactivated at 65°C after ligation was induced, and then a free UMI (NNNANNN) (SEQ ID NO: 28) adapter was spiked in. At this time, the Mock_free adapter did not participate in the ligation process to a free UMI adapter insert, and remained as a free adapter corresponding to the UMI bias. At this time, after finally constructing and sequencing the library, when mock UMI % was confirmed in the same manner, it can be confirmed that the mock UMI % has dropped to 70.18%. These results show that UMI may be introduced up to 30% by functioning as a primer in the PCR process rather than ligation.

At this time, using the double-stranded nucleic acid molecule provided by the present invention and the restriction enzyme, the free adapter was induced to be ligated and then spiked in. If the free adapter was not removed by the double-stranded nucleic acid molecule and the restriction enzyme, according to the experimental results, it was confirmed that the proportion of reads with mock UMI should have been shown as about 70%, but actually shown as 97.74 and 95.25%, which was recovered back to a control level in which mock UMI was added (Mock free adapter linker FR, Mock_free adapter_linker_hairpin).

Through the results, it was confirmed that most of the free adapters have been removed by the double-stranded nucleic acid molecule provided by the present invention and the restriction enzyme, and most of the UMI biases may be removed by using the double-stranded nucleic acid molecule of the present invention.

### INDUSTRIAL APPLICABILITY

According to the double-stranded nucleic acid molecules, the composition, and the method provided by the present invention, only the free adapters in an indexed DNA library for next generation sequencing may be selectively removed to prevent problems of index hopping, UMI mix, and the like caused by the free adapters during the next

## Claims

1. A double-stranded nucleic acid molecule comprising:
a sense strand containing a type IIs restriction enzyme recognition site and a random nucleotide sequence of a length of 3 to 30 nucleotides (nt); and
an anti-sense strand containing a complementary sequence to the sense strand and a 3'-end A tail.

2. The double-stranded nucleic acid molecule of claim 1, wherein a random nucleotide sequence of a length of 1 to 10 nt is further contained in the 3'-end of the sense strand or the 5'-end of the anti-sense strand.

3. The double-stranded nucleic acid molecule of claim 2, wherein the random nucleotide sequence further contained in the 3'-end of the sense strand consists of only cytosine (C), guanine (G), or a combination thereof.

4. The double-stranded nucleic acid molecule of claim 2, wherein a base sequence of the random nucleotide further contained in the 5'-end of the anti-sense strand consists of only thymine (T), cytosine (C), guanine (G) or a combination thereof.

5. The double-stranded nucleic acid molecule of claim 1, wherein the 3'-end of the sense strand and the 5'-end of the anti-sense strand are connected to each other by a loop to have a hairpin structure.

6. The double-stranded nucleic acid molecule of claim 1, wherein the type IIs restriction enzyme is selected from the group consisting of MmeI, FokI, Alw26I, BbvI, BsrI, Earl, HphI, MboI, SfaNI, AlwI, BsaI, BbsI, BbuI, BsmAI, BsmI, BspMI, Esp3I, HgaI, MboII, PleI, SfaNi, Mnll, CspCI, AloI, PpiI, PsrI, BplI, Fall, Bsp24I, BsaXI, HaeIV, CjeI, CjePI, Hin4I, BaeI, AlfI, BcgI, BslFI, and Tth111I.

7. The double-stranded nucleic acid molecule of claim 1, wherein the sense strand comprises a type IIs restriction enzyme recognition site and a random nucleotide of a length of 6 to 10 nt, and the base sequence of the random nucleotide is constituted so that a melting temperature (Tm) value of the double-stranded nucleic acid molecule is 20°C or higher.

8. The double-stranded nucleic acid molecule of claim 1, wherein the sense strand consists of a base sequence defined by SEQ ID NO: 1 and the anti-sense strand consists of a base sequence defined by SEQ ID NO: 2.

9. The double-stranded nucleic acid molecule of claim 5, wherein the double-stranded nucleic acid molecule consists of a base sequence defined by SEQ ID NO: 3.

10. A composition comprising the double-stranded nucleic acid molecule according to any one of claims 1 to 9, DNA ligase, and a type IIs restriction enzyme.

11. The composition of claim 10, wherein the DNA ligase is T4 ligase.

12. The composition of claim 10, wherein the composition is used for improving sample detection ability in an indexed DNA library in next generation sequencing (NGS).

13. The composition of claim 10, wherein the composition is used for removing free adapters which are not ligated to an insert in the process of constructing the indexed DNA library of next generation sequencing.

14. A kit for removing free adapters in an indexed DNA library of next generation sequencing, comprising the double-stranded nucleic acid molecule according to any one of claims 1 to 9, DNA ligase and a type IIs restriction enzyme.

15. A method for removing free adapters in a DNA library for next generation sequencing comprising steps of:
(a) treating and reacting the double-stranded nucleic acid molecule according to any one of claims 1 to 8 and DNA ligase in a DNA library for next generation sequencing constructed by linking adapters for next generation sequencing to both ends of a double-stranded DNA fragment of a genomic DNA to be analyzed; and
(b) treating the resulting reaction products with a type IIs restriction enzyme.

16. The method of claim 15, wherein the adapter comprises an index sequence, a random barcode sequence or both thereof.

17. The method of claim 15, wherein the type IIs restriction enzyme is selected from the group consisting of Mmel, FokI, Alw26I, BbvI, BsrI, Earl, HphI, MboI, SfaNI, AlwI, BsaI, BbsI, BbuI, BsmAI, BsmI, BspMI, Esp3I, HgaI, MboII, PleI, SfaNi, Mnll, CspCI, AloI, PpiI, PsrI, BplI, Fall, Bsp24I, BsaXI, HaeIV, CjeI, CjePI, Hin4I, BaeI, AlfI, BcgI, BsIFI, and Tth111I.

18. The method of claim 15, further comprising:
washing with a buffer or performing bead clean-up after step (b).

19. Use of the double-stranded nucleic acid molecule according to any one of claims 1 to 9, DNA ligase and a type IIs restriction enzyme for preparing a agent for removing free adapters in an indexed DNA library for next generation sequencing.
